# EUROPEAN PATENT APPLICATION

(11) **EP 3 572 034 A1**
(43) Date of publication of application: **27.11.2019**
(21) Application number: 18741659.9
(22) Date of filing: 17.01.2018
(51) Int. Cl.: A61C 8/00, A61C 1/08, A61B 17/17

(54) **TWO-STAGE PRECISION GUIDE AND METHOD FOR FIXING TO A LOW-HEIGHT MAXILLARY BONE A TRIPOD WITH SHORT DENTAL IMPLANT FUNCTION**

(30) Priority: 19.01.2017 ES 201730060
(71) Applicant: Ruesga Delgado, Oscar, 28860 Boadilla del Monte (ES)
(72) Inventor: Ruesga Delgado, Oscar, 28860 Boadilla del Monte (ES)
(74) Representative: Monzón de la Flor, Luis Miguel
(86) International application number: PCT/ES2018/070033
(87) International publication number: WO 2018/134461

(57) **Abstract**

The invention relates to a two-stage precision guide (45) and a method for fixing to a low-height maxillary bone a tripod with short dental implant function, said guide allowing an osteointegrated tripod to be positioned on a low-height maxillary bone in two simple steps. The guide (45) is formed by two elements, which are a fixation guide (1) and a drilling guide (25). In the first stage of use, using the fixation guide (1) adhered to the body (2A) of the tripod, the two-stage guide (45) allows the body (2A) of the tripod, which is characterised in that it contains five perforations, to be positioned on the bone. In the second stage of use, and once the body (2A) of the tripod has been fixed to the bone, the two-stage guide (45) allows the bone to be worked on safely using the drilling guide (25), by coupling the drilling guide to the two elements described above, that is to say, the fixation guide (1) and the body (2A) of the tripod. Once the holes have been made, the two parts of the two-stage guide (45) are removed to be subsequently fixed to the bone by means of the body (2A) of the tripod claimed, which has three identical feet. With the feet in place in the bone, a dental prosthesis can immediately be situated on the tripod when required, the tripod thus acting as a short dental implant

## Description

### Object of the invention

The present invention belongs to the dental implantology sector.
The object of the present invention is a two-stage precision guide and method for fixing to a low-height maxillary bone a tripod with short dental implant function which provides a convenient, quick and safe way to fix a tripod, that can support the load of a prosthesis, in areas of the jaw bone wide but low, avoiding regenerative surgeries such as sinus lift or block maxillary reconstructions.

### BACKGROUND OF THE INVENTION

Short dental implants are implants of relatively recent use. These are wide dental implants, usually greater than 5MM's width and short, usually up to 5MM. These implants are aimed at short and wide areas of bone but, although presenting an acceptable osseointegration, they are very unpredictable when putting a prosthetic load on them, which significantly reduces its use. The Roxolid® SLActive® implant with short Loxim ™ from Straumann and the Anyridge® short implants from IDS are an example of existing short implants. The microimplants are very thin implants of between 1.3MM to 2MM in width and lengths of approximately 10MM whose use is exclusive in orthodontic treatments since they only support tensile forces of up to 300g. The Unitek ™ implant from 3M is an example of an existing microimplant.

There are still many inventions that seek to combine a central dental implant with one or more finer implants that go through it, inventions that seek to provide more stability to the first implant, although none of them provides a concrete, simple, practical solution, feasible application and clinical manipulation of the device, being many of them impossible to perform and impossible to put on the lips or simply unrealistic. In addition, many of them do not provide a universal prosthodontics solution because they carry in their design a pillar for cemented or prosthetic anchoring. Some of these patents I quote them below:
- The patent US2857670A by the author TF Kiernan, Jr with date of publication of October 28, 1958 and US5890902A by the author SAPIAN SL with date of publication of April 6, 1999 both similar, where the authors seek to provide stability to a dental implant by pivots which are perpendicular to the axis of the implant. Besides this implant, is very difficult to perform and unfeasible from a clinical point of view since it would considerably damage the bone and it would be very difficult to remove it if there is any problem.
- The patent US3579831A by the authors IRVING J STEVENS and JERRY ALEXANDER with publication date of May 25, 1971. Here the authors seek to stabilize a dental implant by means of two thin transverse sticks, which lacks clinical and practical sense.
- The Patents: US3981079 (A) of the author LENCZYCKI J J with publication date of September 21, 1976; FR2720264A1 of the authors BOMPARD and MAUDUECH E with date of publication of December 1, 1995; US5542847A from the authors KADAR A and MARGULIES J Y with publication date of August 6, 1996; ATA1212000 by the authors HAAS ROBERT DR and WATZEK GEORG DR with publication date of September 15, 2000; FR2797171A1 by the authors BOMPARD BRUNO JACQUES HUBERT and BOMPARD ELISABETH CATHERINE MA with publication date of 9 February 25, 2001; US2002031747A1, EP1330207A2 of the company ADVANCED DENTAL ENG LTD with publication date of March 14, 2002; FR2848096A1 by author HOFMANN B with publication date of June 11, 2004; FR3006883 (A1) by the authors DADAKARIM and LOUIS JOEL with a submission date of December 19, 2014, all of these patents seek to block and fix an implant by crossing it perpendicularly with a microimplant and providing many of them with technical and clinical use solutions. All these devices are clinically unviable but interesting, although they do not correspond with the patent claimed here.

- The patent KR20110000660A of the author SEVRAIN LIONEL C with publication date of January 4, 2011 tries to provide a practical solution for clinical application, although this solution is very complex and clinically unfeasible since it intends to introduce microimplants in the biological space and the cortical bone area that should not be invaded by a dental implant.
- The patent US2015134016A1 of the company BIEDERMANN TECHNOLOGIES GMBH & CO with publication date of May 14, 2015 that has a purpose other than the one claimed here but seeks to apply a tripod in the bone by inserting sticks around a central pivot to leave them in the bone and anchoring it after bone regeneration surgeries, all this through a practical guide. The guide of this design does not have the indicated precision for the implantology, neither the connection is applicable, besides inserting some micropins or toothpicks that are useless to support prosthodontic loads.
- The patent ES2160498 (A1) of the author HERNANDEZ CABRERA J M with date of publication of November 1, 2001 patent that although ingenious is unworkable from a manufacturing and clinical application point of view.
- The patent WO2009044395 of MEDILOCK MEDICAL SOLUTIONS LTD company with date of publication of April 9, 2009 that seeks to block in the bone a bolt through another, with a very generic description and without any practical solution, this patent is also not focused to the field of oral implantology.
- The patent US5564925 of the author SHAMPANIER A with date of publication of October 15, 1996, although very creative, interesting and with the same purpose as the patent claimed here, this design is technically unviable in all respects since it does not provide the adequate stability for support the prosthodontic occlusal load, does not provide an application technical solution and does not have a
   universal or viable prosthetic solution.
- The patent US3955280 of the author SNEER M with date of publication of May 11, 1976 patent very original but technically unfeasible with a unique prosthetic solution and not universal
- The patent EP3075346 of the author KOSTAKIS G with publication date of June 5, 2016 provides a practical and interesting solution but technically unfeasible since the insertion of the lateral microimplants is done in the biological space and the cortical bone, space that should not be play under no circumstances.
- The patent US5984681 of the author HUANG B K with publication date of November 16, 1999 presents in broad strokes the insertion of micropins in an external connection implant without providing clinical solutions or methodology besides being technically unfeasible in the presentation made.
- The patent CN101947139 by the same author and published on January 19, 2011 presents a simplified but very unstable implant model with a unique, non-universal connection.
- My patent ES1153433 with a filing date of March 10, 2016 and my utility model U201631447 with a filing date of December 18, 2016, are an incomplete and imprecise variant of the patent claimed here, patent that perfects in all the senses the technical realization of the device and simplifies to the maximum its clinical use, presenting some improvements that make it unique as we see below.

### DESCRIPTION OF THE INVENTION

To correct the above-described defects, the invention proposes a simple invention to make and use, realistic, very versatile, ergonomic, respecting established technical standards and very original. The invention is a two-stage precision guide and method for fixing to a low-height maxillary bone a tripod with short dental implant function which is composed of two inseparable and indispensable elements for the realization and comfortable use of it: the fixing guide and the drill guide. These elements must be designed with a geometry and precise measurements for the proper functioning of the device. This two-stage precision guide allows to fix a tripod constituted by a central body and three legs and, as its name indicates, the guide consists of two phases of use.

In the first phase of use of the two-stage precision guide or fixation phase of the tripod's body, we use the fixation guide and the tripod's body, joined the first to the second by a simple cylindrical head's screw that has a narrower cylindrical stem with external helical threads. This screw also has a polyhedral cavity in its head that serves to manipulate it with the appropriate instrument. The fixation guide, whose function is to accurately transport and fix the tripod's body in the low-lying maxillary bone, is a solid block-shaped cup without stem whose foot is polyhedral and whose calyx is cylindrical containing an opened polyhedral cavity. The foot of the guide has five holes. Three of these holes are inclined, they are cylindrical, identical between them and their central axes cross with the central axis of the guide at the same point above the object. These holes do not touch each other and cross the foot completely. The entrance's centres of these holes, located in the base superior of the foot of the guide, draw an equilateral triangle and, the exit's centres of these holes, located in the inferior base at the foot of the guide, draw an equilateral triangle larger than the one described above. Between two of these holes and without touching them, there is a fourth cylindrical hole that's parallel to the central axis of the guide. This fourth hole is narrowed inside by an annular edge perpendicular to the central axis of this hole, this turns it into a new cylindrical hole of smaller diameter and so, the entrance of the fourth hole located in the upper base of the foot of the guide is wider than the exit of it, located in the lower base of the foot of the guide. These four holes surround without touching it a fifth polyhedral hole, perforated from the centre of the upper base of the foot's guide downwards. This hole doesn't cross the foot completely. Above and around this foot is the chalice of the guide which is a cylindrical ring that contains a polyhedral cavity open upwards and delimited from below by the upper base of the foot's guide. They are three rails attached to the outside of this ring that lead the user's guide, as explained later in this description. These are parallel to the central axis of the fixation guide and are each located in one of the three sagittal planes of the three inclined holes mentioned above.

This guide covers the body of the tripod and is fixed by the cylindrical and simple head screw. This body is a solid, cylindrical object with the shape of a wide-floor glass.

This glass contains a polyhedral cavity open upwards, surrounded by a cylindrical wall and superimposed on the floor of the cylindrical floor of the glass. This cavity is the antagonist of the fixation guide's foot. On the outside this wall is smooth and this floor is rough and has external helical threads. Inside this floor is perforated by five perforations that start from the bottom of the glass's cavity, bottom that is also the upper base of the glass's floor. Three of these perforations are inclined, cylindrical, identical between them and their central axes intersect with the central axis of the glass at the same point above the object. These perforations do not touch each other. The entrance's centres of these perforations, located in the bottom of the polyhedral cavity of the glass, draw an equilateral triangle. The outlets of these perforations are located on the sides or at the bottom base of the floor of the glass and their centres also draw an equilateral but larger triangle than the aforementioned one.

These three perforations can be composed of two cylindrical spaces, the upper one wider than the lower, separated by an annular edge perpendicular to the central axis of said perforations. Alternatively, the narrower lower space may contain propeller threads. These three perforations can also be constituted of a single cylindrical space with smooth walls that contain ditches that start from their entrances, that do not touch each other, that are parallel to the central axes of the perforations, that do not cover them in their entirety and which serve to attach the rails of the legs of the tripod on them as we will see later in this description.These two configurations of the three inclined perforations of the tripod's body define two types of bodies, which differ only by this feature. The entrances of these three perforations of the glass have the same width as the exit of the three inclined holes of the fixing guide and serve them as a continuation downwards when the two elements are adhered. This is possible since the entrance and exit's centres of the fixation guide's inclined holes as well as the entrance and exit's centres of the tripod's body inclined perforations draw an overlap of equilateral triangles of increasing size, from top to bottom, when the fixation guide and the glass are tightly joined.

Between two of these glass's inclined perforations and without touching them, there is a fourth cylindrical perforation, parallel to the central axis of the glass and which does not cross its floor completely. This fourth perforation of the glass has the same width as the outlet of the fourth perforation of the fixing guide and serves as a continuation downwards when this guide is inserted in the glass. This fourth perforation of the floor of the glass has a wall containing internal screw threads where the above-mentioned single head screw is screwed. These four perforations surround without touching it a fifth cylindrical perforation, located in the centre of the glass's floor, perforation that does not cross the floor and that is surrounded by a wall with internal helix threads. This is used to attach the correct dental prosthesis to the body of the tripod, using a single head screw. In this first phase of use of the two-stage guide both the fixation guide and the body of the tripod are perfectly joined, the guide's foot inserted inside the glass's cavity and the calyx's lower base of the fixation guide resting on top of the guide.

The arrangement of the three guide rails mentioned above, allows the user to intuit where the tripod's body will be positioned. The user will insert it precisely into the bone, avoiding anatomical areas of risk, either visually or by surgical splint. In the second phase or phase of insertion of the legs of the tripod, once the tripod's body is placed in the bone, by means of the appropriate instrument, the screw that fix the fixing guide to the tripod's body is removed. With the fixing guide still in the tripod's body, we fix the drill guide to the fixing guide end the tripod's body with a long and stepped head screw.

The drill guide is a polyhedral slab with the exact polyhedral shape of the fixing guide's polyhedral cavity where it will be placed. This slab has a polyhedral pivot protruding from the centre of its bottom base downwards. This pivot will correspond to the fifth polyhedral hole of the fixing guide and serves to place the drill guide with precision and stability inside the fixing guide. Above and in a corner of the upper base of the slab is another protruding pivot or handle with polymorphous shape that serves to grip and transport the drill guide. This drill guide contains three inclined, cylindrical and identical holes. Its central axes intersect with the central axis of the drill guide at the same point above this object. These holes do not touch each other and cross the drill guide completely. The centres of the inlets of these holes, located in the upper base of the drill guide, draw an equilateral triangle and, the centres of the outlets of these holes, located in the lower base of the drill guide, draw a larger equilateral triangle. The width of these holes is less than the width of the inclined holes of the fixing guide and of the tripod's body inclined perforations. The outlets of these drill guide's three inclined holes serve as a continuation upwards of the fixing guide's three inclined holes entrances and therefore also of the tripod's body three inclined holes entrances when the three elements are attached. This is possible since the entrances and exits centres of the drill guide's inclined holes, the entrances and exits centres of the fixing guide's inclined holes and the entrances and exits centres of the tripod's body inclined perforations draw a superposition of equilateral triangles of increasing size, from top to bottom, when the drill guide, the fixing guide and the tripod's body are closely joined.

Between two of the drill guide's inclined holes and without touching them, there is a fourth cylindrical hole, parallel to the central axis of the drill guide that cross it completely. This hole is narrowed inside by an annular edge that's perpendicular to the central axis of the hole reducing it to a smaller diameter's hole and so the entrance of the hole, located in the upper base of the drill guide is wider than the hole's exit located at the bottom base of this guide. This outlet also has the same width as the entrance of the fixing guide's fourth hole. To fix the drill guide and the fixing guide perfectly together with the tripod's body, a long-stepped head screw is required. This screw is constituted by two cylindrical volumes superimposed, the upper one being wider, and lower by a cylindrical narrower stem with external threads. This screw also has a polyhedral cavity in its head that serves to manipulate it with the appropriate instrument.

This drill guide allows us to drill narrower holes in the bone than the inclined holes' width of in the tripod's body without touching it. We can also have a set of drill guides that allows to work bone depending of its density and hardness found when drilling. Once the bone has been drilled, the drill guide and fixing guide are removed so that the legs of the tripod can be inserted.

The legs of the tripod have a cylindrical head with a polyhedral cavity in its centre and below, a trunk also cylindrical. We will use the corresponding tripod leg depending on the chosen tripod's body type, thus, for a tripod's body with double width inclined perforations, we will insert some legs with wider heads than their trunks and, for a tripod body with a single space's inclined perforations and ditches near its entrances, we will push some tripod legs with heads of the same width as their trunks, heads that in this case, carry coupling rails parallel to their central axis and polymorphous perforations. These perforations are perpendicular to the central axis of these legs and in contact with their polyhedral cavity and serve to be able to pull these legs if necessary, with the suitable instrument. Three identical legs must be inserted to configure a perfect tripod that distributes loads properly. With the legs already placed on the bone, we can place a prosthesis on the tripod, where appropriate, thus serving this tripod as a short dental implant.

### BRIEF DESCRIPTION OF THE DRAWINGS

The description is supplemented, for an easy understanding of this description, with a set of drawings in which, with an illustrative and non-limiting character, the elements of the invention used during the fixing phase of the tripod body are represented as follows:

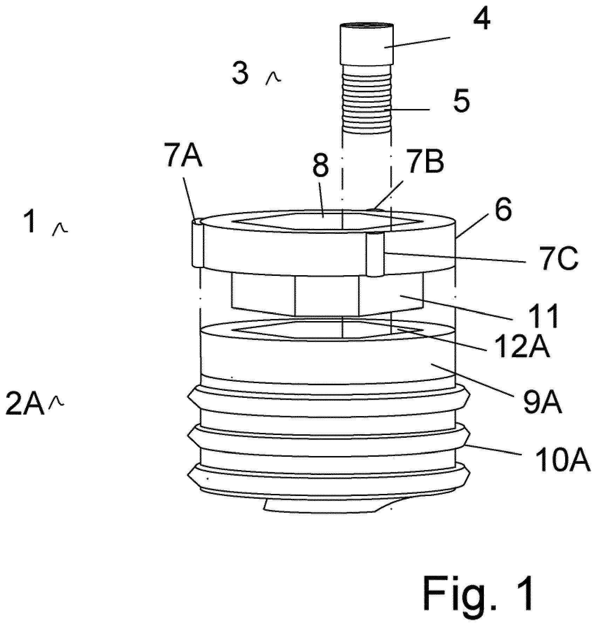

- Figure 1 represents a perspective side view of the two separate elements necessary for the use of the invention in its first phase of use. Here we see above the fixing guide (1) and below the tripod's body (2A) in its first variant. Above the guide (1) we see the screw (3) with simple cylindrical head (4) and stem (5) with external screw threads. This arrangement allows us to see the chalice (6) of the fixation guide (1) with its three guide rails (7A, 7B and 7C) and the polyhedral cavity (8) of the fixation guide (1). Below we see the cylindrical wall (9A) and the cylindrical floor (10A) of the tripod's body (2A). We also see the polyhedral foot (11) of the fixation guide (1) and the polyhedral cavity (12A) of the tripod's body (2A).

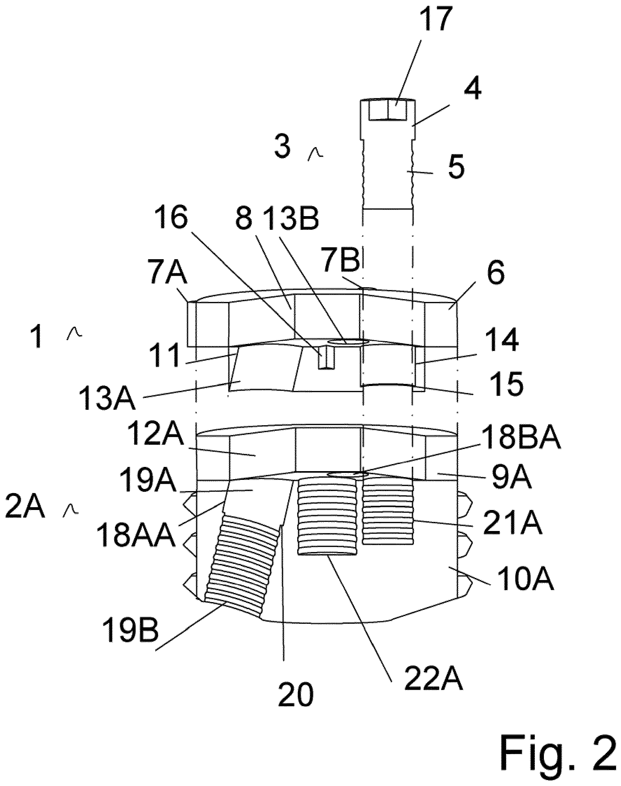

- Figure 2 represents a side view of a sagittal cut in perspective of the two separate elements necessary for the use of the invention in its first phase. Here we see above the fixing guide (1) and below the tripod's body (2A) in the first of its two variants. Above the fixing guide (1) we see the screw (3) with a simple cylindrical head (4) and stem (5) with external screw threads. This arrangement allows us to see, the calyx (6) of the fixation guide (1), a sagittal section of the orientation rail (7A), the orientation rail (7B) and the polyhedral cavity (8) of the fixation guide (1). Below we see the cylindrical wall (9A) and the floor (10A) of the tripod's body (2A) in one of its variants. We also see the polyhedral foot (11) of the fixation guide (1) and the polyhedral cavity (12A) of the tripod's body (2A) in one of its variants. Here we can see more detailed four holes of the fixing guide (1): a hole inclined (13A) in sagittal section, the entrance of another inclined hole (13B), its fourth hole (14) with its annular edge (15) and its fifth polyhedral hole (16). We also see the polyhedral cavity (17) of the screw (3). We also see four perforations of the tripod's body (2A) in one of its variants: an inclined perforation (18AA) represented with a wide upper part (19A) and a narrow lower part (19B) with propelled threads both separated by an annular edge (20); the entrance of another inclined perforation (18BA), its fourth perforation (21A) with propeller threads and its fifth perforation (22A) with propeller threads.

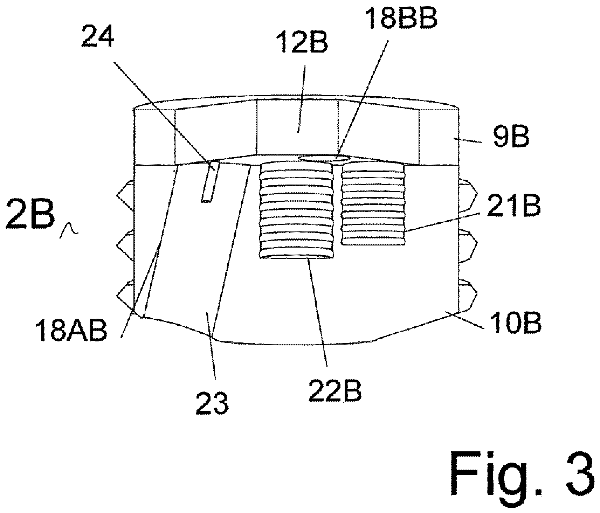

- Figure 3 represents a side view of a sagittal cut in perspective of the second variant of the tripod's body or alternative body (2B), represented with an inclined perforation (18AB) of a single space (23) with a trench (24) on the wall. We also see the polyhedral cavity (12B), the cylindrical wall (9B) and the floor (10B) of the alternative body (2B) of the tripod. We also see, his fourth perforation (21B) with propeller threads and his fifth perforation (22B) with propeller threads.

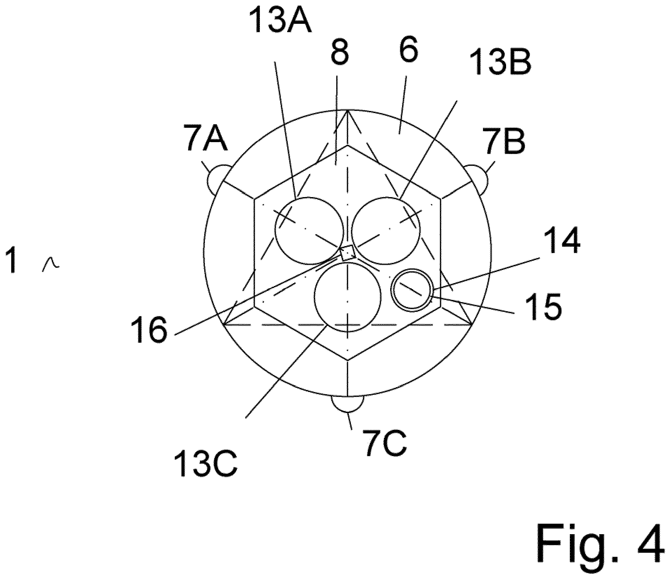

- Figure 4 represents a top view of the fixing guide (1) where we see the chalice (6) of the fixing guide (1) with its three guide rails (7A, 7B and 7C) and the polyhedral cavity (8) of the fixation guide (1). We see the entrances of the three inclined holes (13A, 13B and 13C) of the fixing guide (1), its fourth hole (14) with its annular edge (15) and its fifth polyhedral hole (16).

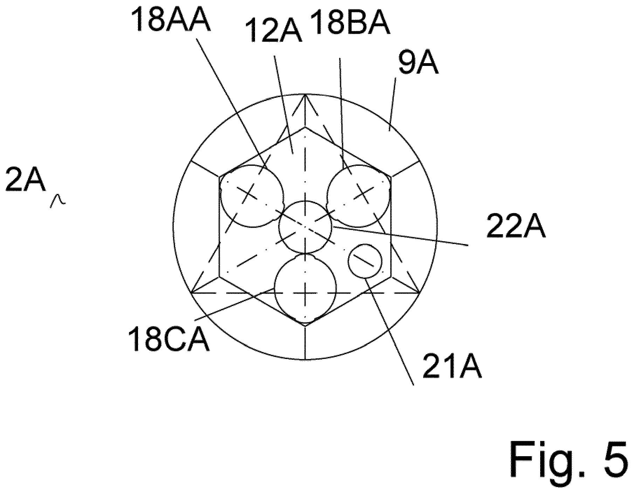

- Figure 5 represents a top view of the first variant of the body's tripod (2A), where we see its cylindrical wall (9A) and its polyhedral cavity (12A). We also see the entrances of the three inclined perforations (18AA, 18BA and 18CA) of the body (2A) of the tripod in one of its variants, its fourth perforation (21A) and its fifth perforation (22A) cylindrical.

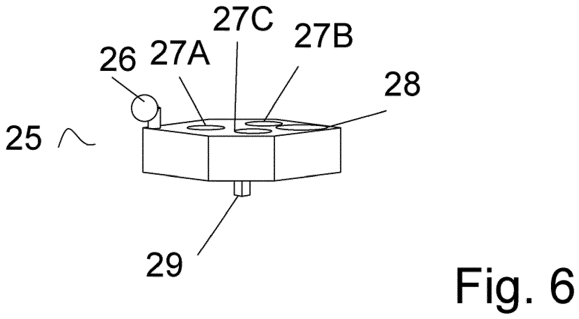

- Figure 6 represents a perspective side view of the drill guide (25) where we can see, the handle (26) of this guide (25) and the four entrances of its four holes: the three entrances of its three inclined holes (27A, 27B and 27C) and the entrance of its fourth hole (28). We can also see the projecting polyhedral pivot (29) of the lower base of this drill guide (25).

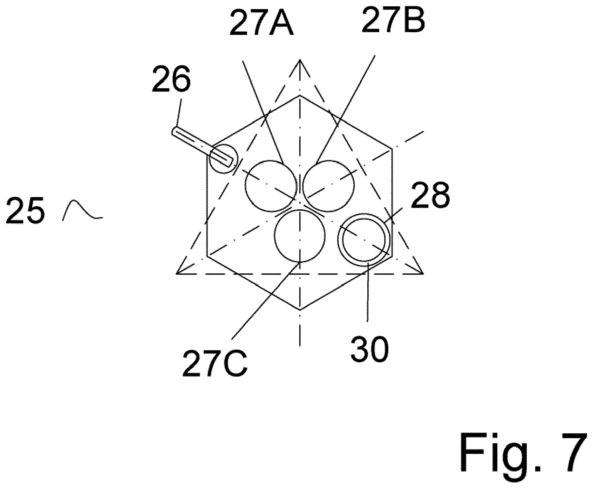

- Figure 7 represents a top view of the drill guide (25) where we see its handle (26) and the four entrances of its four holes: the three entrances of its three inclined holes (27A, 27B and 27C) and the entrance of its fourth hole (28) with its annular edge (30).

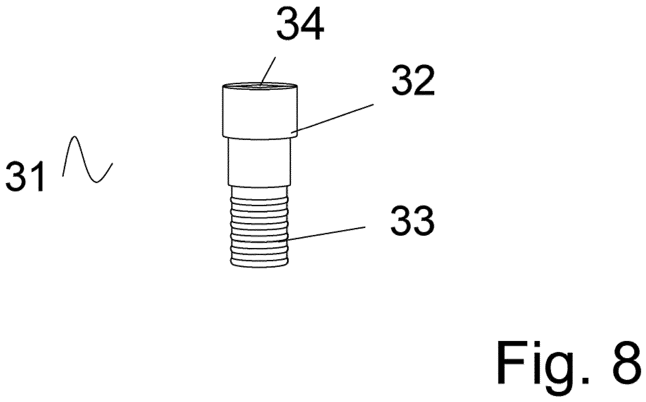

- Figure 8 represents a perspective side view of the long and stepped head (32) screw (31) as well as its stem (33). We also see the polyhedral cavity (34) of the screw (31).

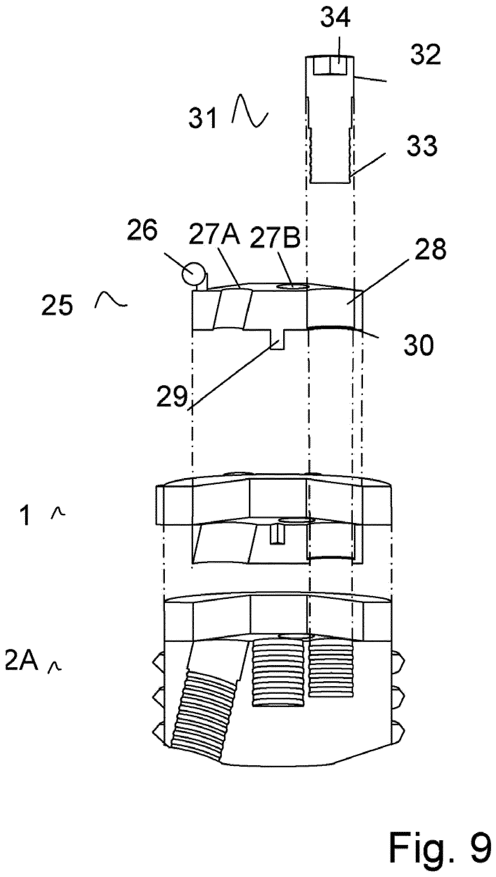

- Figure 9 represents a side view of a sagittal cut in perspective of the three separate elements necessary for the use of the invention in its second phase of use, the fixing guide (1), the first variant of the body (2A) of the tripod and the drill guide (25) together with the corresponding screw (31). Here we see above the drill guide (25) its handle (26), the inclined hole (27A), the entrance of the inclined hole (27B), its fourth hole (28) with its annular edge (30). We can also see the projecting polyhedral pivot (29) of the lower base of this drill guide (25). Above the drill guide (25) we see the long and stepped head (32) screw (31) with its polyhedral cavity (34) as well as its stem (33).

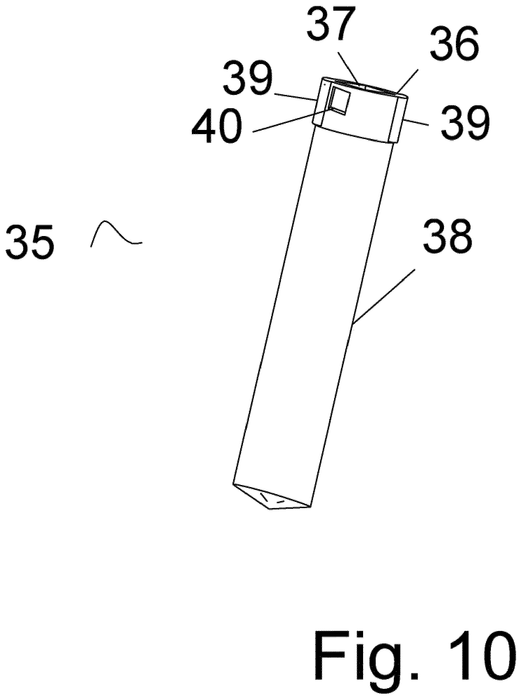

- Figure 10 shows a perspective side view of a possible leg (35) of the tripod here with a cylindrical head (36) having a polyhedral cavity (37) at its centre and a smooth or rough trunk (38). We see the coupling rails (39) of the head (36), here two, and also a polymorphous piercing (40) of the head (36).

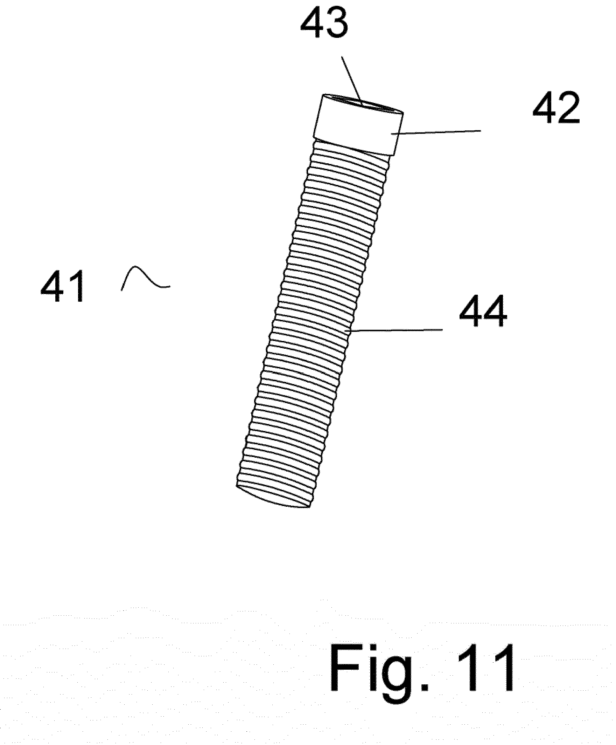

- Figure 11 shows a side perspective view of another possible leg (41) of the tripod here with a cylindrical head (42) having a polyhedral cavity (43) at its centre and a narrower trunk (44) with external helical threads.

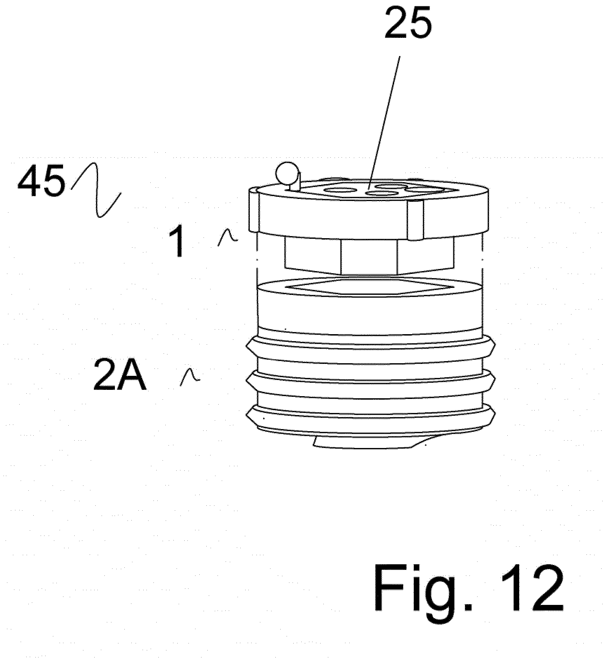

- Figure 12 shows the two stages precision guide (45) for fixing a tripod with short dental implant function with its two elements, the guide (1) for fixing the body (2A) of the tripod in one of its variants and the drill guide (25).

### PREFERENTIAL REALIZATION OF THE INVENTION

As already indicated, and as can be seen in figure 1, in its first phase of use, the two stages precision guide (45) to fix a tripod with short dental implant function, in a low jaw bone , object of the invention, in its preferred embodiment, comprises above the fixing guide (1) and below the body (2A) of the tripod, in the first of its two variants, joined by the simple cylindrical head (4) screw (3) and its stem (5) with external screw threads. More detailed, we see the chalice (6) of the fixation guide (1) with its three guide rails (7A, 7B and 7C) and the polyhedral cavity (8) of the fixation guide (1). Below we see the cylindrical wall (9A) and the cylindrical floor (10A) of the tripod's body (2A) in one of its variants. We also see the polyhedral foot (11) of the fixation guide (1) and the polyhedral cavity (12A) of the tripod's body (2A) in the first of its variants. In figure 2 we see the same objects of figure 1 with a sagittal cut which allows to appreciate in more detail some holes of the fixation guide (1) and of the tripod's body (2A) in the first of its variants: an inclined hole (13A) of the fixation guide in sagittal section, the entrance of another inclined hole (13B), its fourth hole (14) with its annular edge (15) and its fifth polyhedral hole (16). We also see the polyhedral cavity (17) of the screw (3). We also see four perforations of the tripod's body (2A) in the first of its variants: an inclined perforation (18AA) represented with a wide upper part (19A) and a narrow lower part (19B) with helical threads separated by an annular edge (20); the entrance of another inclined hole (18BA), its fourth hole (21A) with propeller threads and its fifth hole (22A) with propeller threads. In figure 3 we see the second variant in a sagittal section of a tripod's body or alternative body (2B), with an inclined perforation of a single space (23) and a trench (24) in its wall. We also see the polyhedral cavity (12B), the cylindrical wall (9B) and the floor (10B) of the alternative tripod's body (2B). We also see, his fourth perforation (21B) with propeller threads and his fifth perforation (22B) with propeller threads. In Figure 4 we see a top view of the fixation guide (1) that allows us to see the proportions of this guide as well as its calyx (6), its four orientation rails (7A, 7B and 7C), its polyhedral cavity (8), the entrances of its three inclined holes (13A, 13B and 13C), its fourth hole (14) with its annular edge (15) and its fifth polyhedral hole (16). In figure 5 we see a top view of the tripod's body (2A) in its first variant, which allows us to see the proportions of this as well as its cylindrical wall (9A), its polyhedral cavity (12A), the entrances of its three perforations inclined (18AA, 18BA and 18CA), its fourth perforation (21A) and its fifth perforation (22A). In figure 6 in its second phase of use, the precision two-stage guide (45) for fixing a tripod with short dental implant function, in a low-lying maxillary bone, object of the invention, in its preferred embodiment, comprises the drill guide (25) with its handle (26) and the four entrances of its four holes: the three entrances of its three inclined holes (27A, 27B and 27C) and the entrance of its fourth hole (28). It also shows the outgoing polyhedral pivot (29) of its lower base. In figure 7 we see a top view of the drill guide (25) where we see its handle (26) and the four entries of its four holes: the three entrances of its three inclined holes (27A, 27B and 27C) and the entrance of its fourth hole (28) with its annular edge (30). In figure 8 we see the long and stepped head (32) screw (31), its polyhedral cavity (34) and its stem (33). In figure 9 we see in more detail the three separate elements necessary for the second phase of use of the invention, the fixing guide (1), the tripod's body (2A) in its first variant, and the drill guide (25) together with the corresponding screw (31). Here we see above the drill guide (25) where we see its handle (26), the inclined hole (27A), the entrance of the inclined hole (27B), its fourth hole (28) with its annular edge (30). We can also see the projecting polyhedral pivot (29) of the lower base of this drill guide (25). Above the drill guide (25) we see the long and stepped head (32) screw (31) with its polyhedral cavity (34) as well as its stem (33). In figure 10 we see a possible leg (35) of the tripod herewith a cylindrical head (36) having a polyhedral cavity (37) at its centre and a smooth or rough trunk (38). We see the coupling rails (39) of the head (36), here two, and also a polymorphous piercing (40) of the head (36). In figure 11 we see another possible leg (41) of the tripod here with a cylindrical head (42) that has a polyhedral perforation (43) at its centre and a trunk (44) with external screw threads. In figure 12 we see the two-stage precision guide (45) for fixing a tripod with dental and short implant function, with its two components the fixation guide (1) and the drill guide (25) fixed to the tripod's body (2A) in the first of its variants.

## Claims

1. Two stage precision guide (45) to fix a tripod with a short dental implant function in a low-lying maxillary bone, is composed of two components: the fixation guide (1) and the drill guide (25). The fixation guide (1) is a solid cup-shaped block without a shaft whose foot (11) is polyhedral and whose chalice (6) is cylindrical that contains a cavity (8) open upwards and polyhedral. The foot (11) of the fixation guide (1) has five holes. Three of these holes (13A, 13B and 13C) are inclined, cylindrical, identical between them and their central axes cross with the central axis of the fixation guide (1) at the same point above the object. These holes (13A, 13B and 13C) do not touch each other and cross the foot (11) completely. The centres of the entrances of these holes (13A, 13B and 13C), located in the upper base of the foot (11) of the fixation guide (1), draw an equilateral triangle. The centres of the outlets of these holes (13A, 13B and 13C), located at the bottom base of the foot (11) of the fixation guide (1) draw a larger sized equilateral triangle. Between two of these holes and without touching them, there is a fourth hole (14) cylindrical and parallel to the central axis of the fixation guide (1). This fourth hole (14) tapers inside by means of an annular edge (15) that is perpendicular to the central axis of this hole, converting it into a new cylindrical and smaller diameter hole. The entrance of the fourth hole (14), located in the upper base of the foot (11) of the fixation guide (1) is wider than its exit that's located in the lower base of the foot (11) of the fixation guide (1). These four holes surround without touching it a fifth polyhedral hole (16), drilled from the centre of the upper base of the foot (11) of the fixation guide (1) downwards without crossing it completely. Above and around this foot there is the chalice (6) of the fixation guide (1) which is a cylindrical ring that is fixed and surrounds above and outside the foot (11) of the fixation guide (1). It contains a polyhedral cavity (8) delimited from below by the upper base of the foot (11) of the fixation guide (1). Adhered to the outside wall of this chalice (6) there are three rails (7A, 7B and 7C), parallel to the central axis of the fixation guide (1). These are each located in one of the three sagittal planes of the three inclined holes (13A, 13B and 13C) mentioned above. The drill guide (25) is a polyhedral slab with the exact polyhedral shape of the polyhedral cavity (8) of the fixation guide (1) where it will be placed. This drill guide (25) has a polyhedral pivot (29) projecting from the center of its bottom base downwards. This pivot (29) will correspond to the fifth polyhedral hole (16) of the fixing guide (1). Above and in a corner of the upper base of the drill guide there is a protruding pivot or handle (26) with polymorphous shape. This drill guide (25) contains three inclined holes (27A, 27B and 27C), cylindrical and identical between them. Its central axes intersect with the central axis of the drill guide (25) at the same point above this object. These holes do not touch each other and go through the guide (25) completely. The centres of the inlets of these holes, located in the upper base of the drill guide (25), draw an equilateral triangle and, the centres of the outlets of these holes, located in the lower base of the drill guide (25), draw a larger equilateral triangle. The width of these holes (27A, 27B and 27C) is less than the width of the inclined holes (13A, 13B and 13C) of the fixation guide (1). The outlets of these three inclined holes of the drill guide (25) serve as a continuation upwards of the entrances of the three inclined holes of the fixation guide (1). Between two of these three drill guide's (25) inclined holes and without touching them, there is a fourth cylindrical hole (28), parallel to the central axis of the drill guide (25) that traverses it completely. This hole (28) tapers inside by means of an annular edge (30) that's perpendicular to its central axis. This edge (30) turns the hole (28) into a new smaller diameter cylindrical hole: the entrance of the hole (28), located in the upper base of the drill guide (25), is wider than the exit of this, located in the lower base of this guide (25). This outlet also has the same width as that of the entrance of the fourth hole (14) of the fixation guide (1).

2. Two-stage precision guide (45) for fixing a tripod with a short dental implant function in a low-lying maxillary bone, according to the first claim, wherein said tripod comprises a central body (2A). This body is a solid, cylindrical object with the shape of a wide-floor glass. This body (2A) contains a polyhedral cavity (12A) open upwards, surrounded by a cylindrical wall (9A) and superimposed on the ground (10A) of that which is cylindrical. On the outside, the wall (9A) is smooth and the floor (10A) of this body (2A) is rough and has external helical threads. Inside, the ground (10A) of this body (2A) is perforated by five perforations that start from the bottom of its cavity (12A), which bottom is also the upper base of the ground (10A) of the body (2A). Three of these perforations (18AA, 18BA and 18CA) are inclined, cylindrical and identical between them, and their central axes intersect with the central axis of the body (2A) at the same point above the object. These perforations do not touch each other. The centers of the entries of these perforations (18AA, 18BA and 18CA), located at the bottom of the polyhedral cavity (12A) of the body (2A), draw an equilateral triangle. The centers of the outlets of these perforations (18AA, 18BA and 18CA) are located on the sides or at the lower base of the ground (10A) of the body (2A), also draw an equilateral but larger triangle than the aforementioned one. These three perforations (18AA, 18BA and 18CA), are composed of two cylindrical spaces (19A and 19B), the upper one (19A) wider than the lower one (19B) separated by an annular edge (20) perpendicular to the central axis of said perforations. The narrower lower space (19B) contains propeller threads. Between two of these inclined perforations of the body (2A) and without touching them, there is a fourth cylindrical perforation (21A), parallel to the central axis of the body (2A) and which does not pass through its floor (10A) completely. This ground's (10A) fourth perforation (21A) of the body (2A) has a wall containing internal helical threads. These four perforations (18AA, 18BA, 18CA and 21A), surround without touching it a fifth cylindrical perforation (22A), located in the centre of the ground (10A) of the body (2A), perforation that does not cross the floor (10A) and that It is surrounded by a wall with internal helix threads.

3. Two-stage precision guide (45) for fixing a tripod with a short dental implant function in a low-lying maxillary bone according to the first claim, wherein said tripod comprises a central alternative body (2B). This alternative body (2B) is a solid and cylindrical object with the shape of a wide-floor glass. This alternative body (2B) contains a polyhedral cavity (12B) open upwards, surrounded by a cylindrical wall (9B) and superimposed on the ground (10B) which is cylindrical. On the outside, the wall (9B) is smooth and the floor (10B) of this alternative body (2B) is rough and has external helical threads. Inside, the floor (10B) of this alternative body (2B) is perforated by five perforations that start from the bottom of its cavity (12B), which is also the upper base of the floor (10B) of the alternative body (2B). Three of these perforations (18AB, 18BB) are inclined and cylindrical, identical between them, and their central axes cross with the central axis of the alternative body (2B) at the same point above the object. These perforations do not touch each other. The entries centres of these perforations (18AB, 18BB), located at the bottom of the polyhedral cavity (12B) of the alternative body (2B), draw an equilateral triangle. The outlets of these perforations (18AB, 18BB) are located on the sides or at the lower base of the ground (10B) of the alternative body (2B) and their centres also draw an equilateral but larger triangle than the aforementioned one. These three perforations (18AB, 18BB) are composed of a single cylindrical space (23) with smooth walls containing ditches (24) that start from their entrances, that do not touch each other, that are parallel to the central axes of the perforations. These ditches (24) do not cover the perforations in their entirety.

4. Method for using the two-stage guide (45) as described in first and second claims, which includes the following steps:
- First, the fixation guide (1) and the tripod's body (2A) are joined. The guide's (1) foot (11) rests inside the cavity (12A) of the body (2A) and the lower base of the calyx's (6) guide (1) rests on the top's base of the body's (2A) tripod's wall (9A).
- Second, the fixation guide (1) and the tripod's body (2A) are fixed with a simple cylindrical head (4) screw (3). This head (4) has a polyhedral cavity (17) and a narrower cylindrical stem (5) with external helical threads. The screw (3) is inserted inside the fourth hole (14) of the fixing guide (1) and the fourth perforation (21A) of the tripod's body (2A).
- Third, the tripod's body (2A) is introduced into the bone and positioned accurately helped by the three rails (7A, 7B and 7C) arranged in the chalice (6) of the fixation guide (1).
- Fourth, the simple cylindrical head's (4) screw (3) is removed.
- Fifth, the drill guide (25), the fixation guide (1) and the tripod's body (2A) are fixed with a screw (31). The screw (31) has a long and stepped head (32) with two cylindrical volumes superimposed, the upper one being wider, and a cylindrical narrower stem (33) with external propeller threads. The screw (31) is inserted into the drill guide's (25) fourth hole (28), into the fixing guide's (1) fourth hole (14) and into the fourth hole (21A) of the tripod's body (2A). This screw also has a polyhedral cavity (34) in its head (32).
- Sixth, the bone is perforated with the appropriate instrument following the inclined holes (27A, 27B and 27C) of the drill guide (25).
- Seventh, the long and stepped head's (32) screw (31) is removed.
- Eighth, the two-stage guide (45) is removed.
- Ninth, the three legs (41) of the tripod are introduced. These legs (41) have a cylindrical head (42) with a polyhedral cavity (43) at its centre and below, a stem (44) also cylindrical and narrower.

5. Method for using the two-stage guide (45) as described in first and third claims, which includes the following steps:
- First, the fixation guide (1) and the tripod's body (2B) are joined. The guide's (1) foot (11) rests inside the body's (2B) cavity (12B) and the lower base of the calyx's (6) guide (1) rests on the top's base of the body's (2B) tripod's wall (9B).
- Second, the fixation guide (1) and the tripod's body (2B) are fixed with a simple cylindrical head (4) screw (3). This head (4) has a polyhedral cavity (17) and a narrower cylindrical stem (5) with external helical threads. The screw (3) is inserted inside the fourth hole (14) of the fixing guide (1) and the fourth perforation (21B) of the tripod's body (2B).
- Third, the tripod's body (2B) is introduced into the bone and positioned accurately helped by the three rails (7A, 7B and 7C) arranged in the chalice (6) of the fixation guide (1).
- Fourth, the simple cylindrical head screw (4) is removed.
- Fifth, the drill guide (25), the fixation guide (1) and the tripod's body (2B) are fixed with a screw (31). The screw (31) has a long and stepped head (32) with two cylindrical volumes superimposed, the upper one being wider, and a cylindrical narrower stem (33) with external propeller threads. The screw (31) is inserted into the drill guide's (25) fourth hole (28), into the fixing guide's (1) fourth hole (14) and into the fourth hole (21A) of the tripod's body (2A). This screw also has a polyhedral cavity (34) in its head (32).
- Sixth, the bone is pierced with the appropriate instrument following the inclined holes (27A, 27B and 27C) of the drill guide (25).
- Seventh, the long and stepped head screw (32) is removed.
- Eighth, the two-stages guide (45) is removed.
- Ninth, the three legs (35) of the tripod are introduced. These legs (35) have heads (36) of the same width as their trunks (38). These heads contain rails (39) that are parallel to their central axis and some polymorphous perforations (40) that are perpendicular to the central axis of these legs (35) and in contact with their polyhedral perforation (37).
